(19) **Europäisches Patentamt / European Patent Office / Office européen des brevets**

(11) **EP 1 592 452 B1**

(12) **EUROPEAN PATENT SPECIFICATION**

(45) Date of publication and mention
of the grant of the patent:
**08.10.2008 Bulletin 2008/41**

(51) Int Cl.:
**A61K 47/24** *(2006.01)*    **A61K 9/113** *(2006.01)*

(21) Application number: **04707534.6**

(22) Date of filing: **03.02.2004**

(86) International application number:
**PCT/EP2004/004526**

(87) International publication number:
**WO 2004/069134 (19.08.2004 Gazette 2004/34)**

(54) **INVERT EMULSION TYPE COMPOSITION CONTAINING AT LEAST ONE ACTIVE AGENT SENSITIVE TO THE PRESENCE OF WATER, AND ITS USES IN COSMETICS AND IN DERMATOLOGY**

UMKEHREMULSION MIT MINDESTENS EINEM GEGENÜBER WASSER EMPFINDLICHEN WIRKSTOFF UND IHRE ANWENDUNGEN IN KOSMETIK UND DERMATOLOGIE

COMPOSITION DE TYPE EMULSION INVERSE CONTENANT AU MOINS UN AGENT ACTIF SENSIBLE A LA PRESENCE D'EAU, ET SON UTILISATION DANS LE DOMAINE COSMETIQUE ET EN DERMATOLOGIE

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **05.02.2003 FR 0301349**

(43) Date of publication of application:
**09.11.2005 Bulletin 2005/45**

(73) Proprietor: **GALDERMA RESEARCH & DEVELOPMENT**
**06410 Biot (FR)**

(72) Inventors:
• **ASTRUC, Fanny**
  **F-06200 Nice (FR)**
• **ORSONI, Sandrine**
  **F-06210 Mandelieu (FR)**
• **BRZOKEWICZ, Alain**
  **F-06600 Antibes (FR)**

(74) Representative: **Allab, Myriam**
**L'OREAL - D.I.P.I.**
**25-29 Quai Aulagnier**
**92600 Asnières (FR)**

(56) References cited:
**EP-A- 0 042 827**    **EP-A- 0 779 071**
**EP-A- 1 097 744**    **WO-A- 02/096571**

## Description

The invention relates to a novel invert emulsion type composition containing at least one active agent sensitive to the presence of water, and its uses in cosmetics and dermatology.

Human skin consists of two compartments, namely a deep compartment, the dermis, and a superficial compartment, the epidermis.

The dermis provides the epidermis with a solid support. It is also its feeder component. It consists mainly of fibroblasts and an extracellular matrix itself composed mainly of collagen, elastin and a substance, called ground substance. Leukocytes, mastocytes or tissue macrophages are also found therein. It also contains blood vessels and nerve fibres.

The epidermis is in contact with the external environment. Its role consists in protecting the body from dehydration and from external attacks, whether they are chemical, mechanical, physical or infectious.

The natural human epidermis is composed mainly of three types of cell, which are the keratinocytes, which are highly predominant, the melanocytes and the Langerhans' cells. Each of these cell types contributes by its specific functions to the essential role played in the body by the skin.

The cells constituting the epidermis are delimited by a lipid domain. The epidermal lipids are synthesized mainly in the living epidermis. They consist mainly of phospholipids, sphingolipids, cholesterol, free fatty acids, triglycerides, cholesterol esters and alkanes. During cell differentiation, the phospholipids, whose role consists in producing the fluid structure of the cell membranes of the living layers of the epidermis, are gradually replaced by a mixture mainly composed of fatty acids, cholesterol and sphingolipids, the essential constituents of the horny layer of the epidermis (stratum corneum).

The lipids of the intercorneocyte cement of the skin, and in particular the ceramides, are organized into lamellar bilayers or sheets and participate in the cohesion of the stratum corneum in order to maintain the integrity of the barrier and its protective, anti-penetration and anti-irritation role in particular.

Numerous active agents exhibit the difficulty of being very sparingly soluble in the commonly used cosmetic or pharmaceutical solvents, in particular water, and sensitive to an aqueous environment. This sensitivity to water can lead to chemical instability of the active agent and/or to crystallization of the active agent initially solubilized. This sensitivity to water therefore limits their formulation into cosmetic or dermatological compositions applied by the topical or oral route.

The phenomena of chemical degradation and/or crystallization of the active agent in the presence of water have as consequence a loss of efficacy and an uncertainty as regards the dose of active agent used during its use, which runs counter to the desired objective. In addition, this degradation of the active agent and/or its crystallization can modify the overall stability of the compositions and their appearance.

The expression active agent sensitive to the presence of water according to the invention is understood to mean active agents such as those chemically and/or physically unstable. For example, by chemically unstable, it may be understood that the active agent deteriorates in the composition. For example, by physically unstable, it may be understood that the active agent crystallizes or precipitates in the composition.

The galenic form currently most commonly used in dermatology is the oil-in-water emulsion in which the active agent is preferably solubilized in the lipophilic phase. However, this solution remains scarcely satisfactory because to respond to an objective of an active agent concentration having a quantifiable therapeutic efficacy would require very high concentrations of solvent oils, leading to products which are undoubtedly not very pleasant to use, due to their sticky feel, and which are physically unstable while retaining a limited active agent concentration.

Moreover, the solubilization of the active agent in the internal phase of the emulsion limits its release, the external aqueous or hydroglycolic phase constituting, for the active agent, a physical barrier to its release and its diffusion towards the skin layers.

Another possibility is to solubilize the active agent in the external hydrophilic phase of the emulsion, within the limit of its solubility in the aqueous or hydroglycolic media. However, this solution does not make it possible to solve the problems of chemical stability encountered, because the water activity of the emulsion remains very high.

The replacement of all or part of the aqueous phase with one or more glycols would lead to formulations which are cosmetically not very acceptable. It is indeed known to persons skilled in the art that above 20% glycol, the formulation is cosmetically not very acceptable by virtue of its sticky feel, and its physical stability would not be guaranteed.

The production of an invert emulsion (the expression invert emulsion is understood to mean an emulsion of the hydrophilic phase dispersed in lipophilic phase type) as an alternative was not obvious to persons skilled in the art, given the known difficulties of formulating active agents exhibiting problems of chemical instability and/or crystallization from water.

The patent application EP 0 779 071 describes triple emulsions water-in-oil-in-water comprising an agent sensitive to the presence of water.

The use of hydrophilic solubilizing agents such as propylene glycol was also not natural for persons skilled in the art, given that the high concentrations necessary were not favourable to a good physical stability of the formula and to an acceptable cosmetic feel.

The obtaining of good tolerance with solubilizing agents such as propylene glycol was also not obvious because phenomena of skin intolerance had been shown in humans, for example in healthy humans (Motoyoshi et al., Cosmet. and toiletries, 99, 83-89, 1984).

A need therefore existed for a composition which makes it possible to respond to one or more of the following aspects: to have good stability of the formula to cold and to heat, in particular as regards maintaining the size of the globules and the absence of phase separation, to have good resistance of the active agent to the phenomena of oxidation, to allow good chemical stability of the active agent and good availability thereof for the skin, to exhibit good skin tolerance. It is also useful to be able to have a composition allowing a high dispersed volume fraction. It is moreover useful for the preparation of such compositions to benefit from an advantageous mode of preparation.

Now, the Applicant has surprisingly developed a formulation of the glycol-in-oil type which makes it possible to dispense with the various problems linked to the abovementioned aspects while making it possible in particular to have good physical stability of the composition as such but also to allow good chemical stability and availability of the active agent which it contains. The composition according to the invention also has the advantage of exhibiting good skin tolerance and allowing a high dispersed volume fraction.

The invention therefore relates to a composition containing at least one active agent sensitive to the presence of water, characterized in that the composition is an invert emulsion containing a glycolic or hydroglycolic dispersed hydrophilic phase, a lipophilic continuous phase and an emulsifier having an HLB of between 2 and 7.

The term HLB is understood to mean the Hydrophilic/Lipophilic Balance (HLB), which corresponds to the balance between the size and strength of the hydrophilic group and the size and strength of the lipophilic group of the emulsifier.

The invention also makes it possible to obtain good release/penetration of the active agent at the level of the various skin layers, leading to good availability of the active agent in the skin, the said active agent being used in solubilized form.

The expression solubilized form is understood to mean a dispersion in the molecular state in a liquid, no crystallization of the active agent being visible with the naked eye or even under a cross-polarization optical microscope.

The formulation of the solubilized active agents in a glycolic or hydroglycolic phase into an invert emulsion according to the invention thus makes it possible, surprisingly, to dispense with the problems of chemical stability and crystallization commonly encountered in formulation with the type of active agent used according to the invention.

The present invention therefore consists in preparing invert emulsions, containing a glycolic or hydroglycolic hydrophilic phase, which are perfectly stable (size of the globules and viscosity), even at a high dispersed volume fraction, showing no chemical degradation and/or crystallization of the active agent.

The present invention also relates to the preparation of invert emulsions containing an active agent as defined in claim 1 solubilized in the lipophilic phase of the emulsion, and exhibiting good physicochemical stability, and no crystallization of the active agent.

The preferred active agents according to the invention are the derivatives of vitamin D.

1,25-dihydroxyvitamin $D_3$ (calcitriol), and (4E,6E)-7-[3-(3,4-bishydroxymethylbenzyloxy)phenyl]-3-ethylnona-4,6- dien-3-ol and {4-[6-Ethyl-4'-(1-éthyl-1-hydroxy-propyl)-2'-propyl-biphényl-3- yloxyméthyl]-2-hydroxyméthyl-phényl)-methanol.

which are described and claimed by the applicant in patent application WO 00/26167.

The composition according to the invention is preferably suitable for topical application to the skin, the superficial body growths and/or the mucous membranes. It generally contains a physiologically acceptable medium and a sufficient quantity of active compound to obtain the desired effect. The proportion by weight of active agent, relative to the total weight of the composition, may thus be between 0.001% and 20% (weight/weight), for example between 0.1 and 20%, in particular between 0.2 and 10%, especially between 0.2 and 4%, for example between 0.2 and 2%.

The glycols to be considered in the present invention may be defined as alkylene or polyalkylene glycols. By way of examples, there may be mentioned alkylene and polyalkylene glycols (C1 to C6) such as ethylene glycol, polyethylene glycol (2 to 20 monomers), propylene glycol, dipropylene glycol, butylene glycol, pentylene glycol and hexylene glycol. They may be oxyethylenated or otherwise (2 to 50 EO). Those preferred according to the invention are hexylene glycol, propylene glycol and dipropylene glycol, and polyethylene glycol 400 (PEG 400).

The glycols which can be used according to the invention will advantageously have, as solubility parameter, a $\delta p$ of less than 10, it being understood that the 3 Hansen solubility parameters - $\delta d$, $\delta p$ and $\delta h$ - characterize, for a given constituent, the energies corresponding respectively to the dispersive, polar and hydrogen bond type interactions existing between the molecules of this constituent, $\delta p$ characterizing more particularly the Debye forces of interaction between dipoles and being a function of the number of oxygen atoms in the formula of the given constituent (S. paint Technology, 30, 195, 1967, "The three dimensional solubility parameter - Key to paint component affinities").

As lipophilic compounds which can be used to constitute the continuous fatty phase of the emulsions according to the invention, there may be mentioned mineral oils (paraffin oil), oils of plant origin (avocado oil, soya-bean oil), oils of animal origin (lanolin), synthetic oils (perhydrosqualene), silicone oils (cyclomethicone, dimethicone) and fluorinated oils (perfluoropolyethers). It is also possible to use fatty alcohols such as cetyl alcohol, Guerbet alcohols, in particular octyldodecanol known under the name Eutanol G, fatty acids, waxes, gums and in particular silicone gums.

The fatty phase may also consist of linear or branched mono-, di- or triesters of synthetic origin, in particular isopropyl myristate or palmitate, or caprylic/capric triglyceride (Miglyol 812).

Preferably, nonoxidizable compounds are used to constitute the oils of the continuous lipophilic phase, which are preferably chosen from those of the silicone type, those of the ester type or those of the mineral type.

The compounds entering into the composition of the lipophilic phase of the emulsion will have as Hansen solubility parameter a $\delta p$ of less than 5, and for example of between 0 and 2.

Moreover, in order to avoid any crystallization of the active ingredient, the overall solubility parameter for the lipophilic phase - $\delta t_t = \sqrt{\delta d} + \delta p + 8h$ - will have a value of less than 20, for example of between 10 and 20, and preferably of between 12 and 18.

The volume fraction of the dispersed hydrophilic phase in the emulsion according to the invention ranges from 10 to 90% relative to the total volume of the emulsion. It may be exclusively glycolic or hydroglycolic. The volume proportion of glycols (relative to the total volume of the dispersed phase) is between 10 and 100%, for example between 30 and 100%, in particular between 60 and 100%, and preferably between 80 and 100%.

For a cosmetic application, between 30 and 50% of glycols will be preferably used (a proportion relative to the total volume of the dispersed phase).

It is also possible to characterize a preferred embodiment of the invention with reference to the water activity $(a_w)$ of the hydrophilic phase in the composition according to the invention.

The invention thus also relates, specifically, to a composition as defined above, characterized in that the water activity $a_w$ of the hydrophilic phase is less than 0.85.

The water activity $a_w$ of a medium containing water is the ratio of the vapour pressure of water in the product "$P_{H2O}$ product" and the vapour pressure of pure water "$P_{H2O}$ pure" at the same temperature. It may also be expressed as the ratio of the number of water molecules "$N_{H2O}$" to the total number of molecules "$N_{H2O} + N_{dissolved\ substances}$", which takes into account those of the dissolved substances "$N_{dissolved\ substances}$".

It is given by the following formulae:

$$a_w = \frac{P_{H2O}\ product}{P_{H2O}\ pure} = \frac{N_{H2O}}{N_{H2O} + N_{dissolved\ substances}}$$

It is possible to use various methods for measuring the water activity $a_w$. The most common is the manometric method by which the vapour pressure is measured directly.

Conventionally, a cosmetic or dermatological composition has a water activity of around 0.95 to 0.99. A water activity of less than 0.85 represents a substantial reduction.

Emulsifiers (or surfactants) are natural or synthetic substances consisting of a hydrophilic or polar part and a lipophilic or apolar part. They are amphiphilic molecules since they have a double polarity. Emulsifiers are characterized by their HLB; if the HLB is high, the hydrophilic part is predominant, if the HLB is low, the lipophilic part predominates.

Among these emulsifiers are preferably included polymeric emulsifiers which are characterized by a high molar mass and a nonlinear structure which allows greater anchorage at the water/oil interface than that obtained with the monomer-type emulsifiers.

The emulsifiers which it is possible to use according to the invention, alone or as a mixture, are those which make it possible to make invert emulsions having an HLB of less than 7.

In general, the preferred emulsifiers are the silicone emulsifiers, of the organopolysiloxane type, such as:

- E1)polyalkyl methicone copolyols (oxyalkylenated, optionally crosslinked polyalkyl methylsiloxanes) containing:

    linear or branched, saturated or unsaturated, C6 to C20 alkyl chains

    a polyoxyethylenated unit of 1 to 50 EO (ethylene oxide)
    and/or

    a polyoxypropylenated unit of 1 to 50 PO (propylene oxide) - E2) oxyalkylenated polyalkyl dimethyl methylsiloxanes containing:

    linear or branched, saturated or unsaturated, C6 to C20 alkyl chains
    a polyoxyethylenated unit of 1 to 50 EO

and/or
a polyoxypropylenated unit of 1 to 50 PO

The organopolysiloxanes of the composition of the invention contain in particular one or more oxyalkylenated and in particular oxyethylenated (EO) groups, for example from 1 to 40 oxyalkylenated units, preferably from 1 to 20, even better from 10 to 20, more preferably from 12 to 20 and better still from 12 to 18 oxyalkylenated units, which can form polyoxyalkylenated and in particular polyoxyethylenated chains. These groups may be pendant or at the chain end. The silicon atoms carrying these groups are advantageously about 1 to 10 and even better 1 to 6 in number. The silicone structure forming the polymeric backbone of the organopolysiloxane containing (an) oxyalkylenated group(s) is advantageously a polydimethylsiloxane (PDMS) structure of which some of the methyl groups are optionally substituted with C2 to C30, and preferably C8 to C24, and even better from C10 to C20, alkyl groups or phenyl groups, at the chain end or pendant.

Advantageously, there will therefore be used as E1 or E2 type emulsifiers silicone emulsifiers such as alkyl dimethicone copolyols such as Abil EM-90, or the dimethicone copolyol and cyclomethicone mixture, sold by the company Dow Corning under the name 3225C Formulation Aid, lauryl methicone copolyol sold under the name Emulsifier 10 by Dow Corning, or mixtures based on a silicone polymer such as cetyl dimethicone copolyol with polyglyceryl-4 isostearate and hexyl laurate sold under the name Abil WE09 by the company Goldschmidt, Abil EM97 from Goldschmidt (dimethicone copolyol & cyclomethicone), Wacker SPG 128 VP from Wacker (cyclomethicone and octyl dimethicone methoxy glycosyl), or alternatively Silwax WD-IS (dimethicone copolyol isostearate).

E3) Mono- or polyalkyl ester siloxanes, for example Silwax S from Lambent (dimethiconol stearate),
E4) alkoxylated carboxylic acid esters such as polyhydroxylated alkyl esters of PEG, for example Arlacel P 135 from Uniqema (PEG-30 dipolyhydroxystearate).

There will be preferably used emulsifiers having an HLB of between 2 and 7, preferably a silicone W/O emulsifier having an HLB of between 2 and 7, preferably a polymeric silicone W/O emulsifier having an HLB of between 2 and 7.

The invert emulsion of the invention may be, as a variant, produced and stabilized with emulsifiers or with the following combinations with an emulsifying character:

1) the combination of an oxyalkylenated crosslinked elastomeric organopolysiloxane and a crosslinked and at least partially neutralized poly(2-acrylamido-2-methylpropanesulphonic acid) polymer.

The composition according to the invention will contain in particular, expressed as a percentage by weight, from 0.5 to 8% of emulsifier, for example from 0.5 to 5%, preferably between 3 and 5%, relative to the total weight of the composition.

Moreover, advantageously, to improve the stability of the dispersion, it is possible to combine the principal emulsifiers described above with one or more coemulsifiers having an HLB of greater than 6. The (coemulsifier/emulsifier) ratio will be advantageously less than 1.5, and preferably less than 0.75.

By way of example, there may be mentioned:

- polyoxyethylenated or nonpolyoxyethylenated alkyl or polyalkyl esters of sorbitan with between 1 and 5 branched or unbranched, saturated or unsaturated C10 to C20 alkyl chains, and with from 0 to 40 EO (for example sorbitan monolaurate 20 EO or sorbitan monooleate 20 EO (Tween 80 from Uniqema));
- polyoxyethylenated alkyl or polyalkyl ethers or esters with between 1 and 5 branched or unbranched, saturated or unsaturated C10 to C20 alkyl chains and with 0 to 40 EO (ceteareth-20 (Eumulgin B2 from Cognis), or steareth (Brij 78) 20 EO);
- ethoxylated and esterified alkyl or polyalkyl mono- or polyglucosides with between 1 and 5 branched or unbranched, saturated or unsaturated C6 to C20 alkyl chains and from 1 to 10 glucose units (for example PEG 20 methyl glucose sesquistearate (Glucamate SSE-20 from Amerchol));
- polyglycerol alkyl or polyalkyl esters or ethers with between 1 and 5 branched or unbranched, saturated or unsaturated C10 to C20 alkyl chains and from 1 to 8 glycerol units (for example polyglyceryl-4 isostearate or PEG-8 stearate (Myrj 45)).

Finally, it is possible to advantageously add to the dispersed phase from 0 to 10% by weight, relative to the total weight of the formulation, of a cosolvent for the active agent having an evaporation temperature of less than 100°C, preferably linear or branched C1 to C4 alcohols, such as ethanol and isopropanol.

Advantageously, the preparation of the emulsion according to the invention was found to require only little mechanical or thermal energy compared with the preparations of other invert emulsions already known.

In a known manner, the composition of the invention may also contain the adjuvants customarily used in the cosmetic and dermatological fields, such as hydrophilic or lipophilic gelling agents, humectants such as glycerine and sorbitol, fatty phase thickeners, preservatives, antioxidants, electrolytes, solvents, perfumes, fillers, screening agents, pigments, odour absorbers, colouring matter and metal-chelating agents. The quantities of these various adjuvants are those conventionally used in the fields considered, and are for example from 0.01 to 20% of the total weight of the composition. These adjuvants, depending on their nature, may be introduced into the lipophilic phase or into the hydrophilic phase. These adjuvants, and their concentrations, should be such that they do not adversely affect the cosmetic and/or dermatological properties of the composition according to the invention.

As hydrophilic gelling agents, there may be mentioned in particular carboxyvinyl polymers (carbomer), acrylic copolymers such as acrylate/alkyl acrylate copolymers, polyacrylamides, polysaccharides, natural gums and clays, and, as lipophilic gelling agents, there may be mentioned modified clays such as bentones, metal salts of fatty acids and hydrophobic silica.

The composition according to the invention has a cosmetically acceptable feel, good skin tolerance, good physical stability, that is to say absence of phase separation and retention of the size of the globules in the cold (at 4°C) and in heat (45°C) over a long period, for example over 2 months, with a stable viscosity over this period. The composition according to the invention also makes it possible to confer on the active agent good chemical stability and to avoid its crystallization over time.

In particular, the invention relates to a cosmetic or dermatological composition for topical application to the skin, the superficial body growths and/or the mucous membranes, in the form of an invert emulsion containing a dispersed glycolic or hydroglycolic hydrophilic phase and a lipophilic continuous phase, characterized in that it contains, in a physiologically acceptable medium (that is to say compatible with topical application to the skin, the superficial body growths and/or the mucous membranes), expressed as a percentage by weight:

- from 0.001 to 5% of at least one active agent sensitive to the presence of water and more particularly of a derivative of vitamin D,
- from 30 to 100% of glycols,
- from 0.5 to 8% of emulsifier having an HLB of between 2 and 7,
- from 0% to 5% of coemulsifier having an HLB of greater than 6,
- from 0 to 50% of water, for example from 0 to 20% of water.

In a particular embodiment of the invention, the dispersed hydrophilic phase has a water activity of less than 0.85.

The invention also covers the use of the novel invert emulsion as described above in cosmetics and in dermatology.

By virtue of the activity of the preferred compounds used in the composition, the composition according to the invention finds application in the prevention and/or treatment of the following pathologies:

1) for treating dermatological conditions linked to a keratinocyte or sebocyte differentiation or proliferation disorder, in particular for treating acne vulgaris, comedo-type acne, polymorphic acne, acne rosacea, nodulocystic acne, acne conglobata, senile acne, secondary acne such as solar acne, acne medicamentosa or occupational acne;

2) for treating keratinization disorders, in particular ichthyosis, ichthyosiform states, Darier's disease, keratosis palmaris et plantaris, leukoplakia and leukoplakia-like states, skin or mucosal (buccal) lichen;

3) for treating other dermatological conditions linked to a keratinization disorder with an inflammatory and/or immunoallergic component, and in particular all forms of psoriasis, whether cutaneous, mucosal or ungual, and even arthropathic psoriasis, or skin atopy, such as eczema, or respiratory atopy or gingival hypertrophy;

4) for treating certain inflammatory skin conditions not exhibiting keratinization disorder, such as atopic eczema and contact allergies;

5) for treating all dermal or epidermal proliferations, whether benign or malignant, whether of viral origin or otherwise, such as verruca vulgaris, verruca plana and epidermodysplasia verruciformis, oral or florid papillomatosis and proliferations which may be induced by ultraviolet radiation, in particular in the case of baso- and spinocellular epithelioma;

6) for treating other dermatological disorders such as bullous dermatosis and collagen diseases;

7) for preventing or treating the signs of skin ageing, whether photoinduced or chronological, or for reducing actinic pigmentations and keratoses, or any skin pathologies associated with chronological or actinic ageing;

8) for preventing or treating cicatrization disorders or for preventing or repairing stretch marks;

9) for combating sebaceous function disorders such as acne hyperseborrhoea or ordinary seborrhoea or seborrhoeic eczema;

10) for treating certain ophthalmological disorders, in particular corneopathies;

11) for the treatment or prevention of the cancerous or precancerous states of skin or nonskin cancers exhibiting or capable of being induced to exhibit vitamin D receptors, such as, but without limitation, breast cancer, leukaemia, myelodysplastic syndromes and lymphomas, carcinomas of the cells of the malpighian epithelium and gastrointestinal cancers, melanomas, and osteosarcoma;

12) for the treatment of inflammatory conditions such as arthritis or rheumatoid arthritis;

13) for the treatment of any condition of viral origin at the cutaneous or general level;

14) for the prevention or treatment of alopecia of various origins, in particular alopecia due to chemotherapy or to radiation;

15) for the treatment of dermatological or general conditions with an immunological component;

16) for the treatment of immunological conditions, such as autoimmune diseases (such as, but without limitation, type 1 diabetes mellitus, multiple sclerosis, lupus and lupus-type conditions, asthma, glomerulonephritis, etc.), selective dysfunction of the immune system (for example AIDS) and the prevention of immune rejection, such as graft rejections (for example the kidney, the heart, the bone marrow, the liver, pancreatic islets or the whole pancreas, the skin, etc.) or the prevention of graft-versus-host disease;

17) for the treatment of endocrine conditions which can be treated with vitamin D analogues such as those advantageously modulating hormone secretion, for example, by increasing the secretion of insulin or selectively suppressing the secretion of the parathyroid hormone (for example in chronic renal insufficiency and secondary hyperparathyroidism);

18) for the treatment of conditions characterized by an abnormal management of intracellular calcium; and

19) for the treatment or prevention of vitamin D deficiency and other conditions related to mineral homeostasis in the plasma and the bones, such as rickets, osteomalacia, osteoporosis, in particular in the case of menopausal women, renal osteodystrophy, and parathyroid function disorders.

The compounds according to the invention also find application in the cosmetic field, in particular in body and hair hygiene and in particular for the treatment of skins with a tendency to develop acne, for hair regrowth or for slowing its loss, for combating the greasy appearance of the skin or the hair, in protecting against the harmful effects of the sun or in the treatment of dry skins, for preventing and/or treating photoinduced or chronological skin ageing.

The invention also covers the pharmaceutical preparations and the medicaments obtained from the compositions according to the invention.

The present invention therefore relates to a composition containing at least one active agent sensitive to the presence of water, which is not DHEA and/or its chemical and/or biological precursors or derivatives, characterized in that the composition is an invert emulsion containing a glycolic or hydroglycolic dispersed hydrophilic phase, a lipophilic continuous phase and an emulsifier having an HLB of between 2 and 7.

The composition according to the invention is characterized in that the active agent is chosen from the group consisting of a synthetic retinoid, a derivative of vitamin D and a derivative of minoxidil, and preferably a derivative of vitamin D, (4E,6E)-7-[3-(3,4-bishydroxymethylbenzyloxy)phenyl]-3-ethylnona-4,6-dien-3-ol and {4-[6-Ethyl-4'-(1-éthyl-1-hydroxy-propyl)-2'-propyl-biphényl-3-yloxyméthyl]-2-hydroxyméthyl-phényl}-methanol.

The composition according to the invention is characterized in that it also contains one or more active agents chosen from isoflavonoids, metalloproteinase inhibitors, carotenoids, anti-glycation compounds, NO-synthase inhibitors, vitamins, desquamating agents, compounds increasing the synthesis of glycosaminoglycans, anti-irritant compounds, compounds reducing irritation of neurogenic origin, muscle-relaxing compounds and depigmenting agents.

The composition according to the invention is characterized in that it contains between 0.001 and 20% by weight of the active agent sensitive to the presence of water, relative to the total weight of the composition, and more particularly the composition contains between 0.01 and 4% by weight of derivatives of vitamin D, relative to the total weight of the composition.

The composition according to the invention is characterized in that the emulsifier is a silicone emulsifier, chosen from lauryl methicone copolyol, cetyl dimethicone copolyol, a mixture of dimethicone copolyol and cyclomethicone or a mixture of cetyl dimethicone copolyol with polyglyceryl-4 isostearate and hexyl laurate.

The composition according to the invention is characterized in that it also contains a coemulsifier having an HLB of greater than 6, which is preferably ceteareth-20.

In the compositions according to the invention, the proportion by volume of glycol, relative to the total volume of the dispersed phase, is between 10 and 100%, the glycol being preferably chosen from propylene glycol, hexylene glycol, dipropylene glycol and PEG 400.

The composition according to the invention is characterized in that the water activity of the dispersed hydrophilic phase is less than 0.85.

The invention also relates to a cosmetic or dermatological composition for topical application to the skin, the superficial body growths and/or the mucous membranes, in the form of an invert emulsion containing a dispersed glycolic or hydroglycolic hydrophilic phase and a lipophilic continuous phase, characterized in that it contains, in a physiologically acceptable medium, expressed as a percentage by weight:

- from 0.001 to 5% of an active agent sensitive to the presence of water, other than DHEA and/or its chemical and/or biological precursors or derivatives,
- from 30 to 100% of glycols,
- from 0.5 to 8% of an emulsifier having an HLB of between 2 and 7,
- from 0% to 5% of a coemulsifier having an HLB of greater than 6,
- from 0 to 50% of water, for example from 0 to 20% of water.

The invention relates to the use of the composition according to the invention for the prevention or treatment:

- of dermatological conditions linked to a keratinocyte or sebocyte differentiation or proliferation disorder;
- of keratinization disorders;
- of dermatological conditions linked to a keratinization disorder with an inflammatory and/or immunoallergic component;
- of inflammatory skin conditions not exhibiting a keratinization disorder;
- of dermal or epidermal proliferation;
- of dermatological disorders such as bullous dermatosis and collagen diseases;
- of the signs of skin ageing, whether photoinduced or chronological, or for reducing actinic pigmentations and keratoses, or any skin pathologies associated with chronological or actinic ageing;
- of cicatrization disorders and stretch marks;
- of sebaceous function disorders such as acne hyperseborrhoea or ordinary seborrhoea or seborrhoeic eczema;
- of dermatological conditions with an immunological component.

More particularly, the invention relates to the use of a composition according to the invention for the prevention or treatment of acne vulgaris, comedo-type acne, polymorphic acne, acne rosacea, nodulocystic acne, acne conglobata, senile acne, secondary acne such as solar acne, acne medicamentosa or occupational acne, ichthyosis, ichthyosiform states, Darier's disease, keratosis palmaris et plantaris, leukoplakia and leukoplakia-like states, skin or mucosal (buccal) lichen, various forms of psoriasis, whether cutaneous, mucosal or ungual, arthropathic psoriasis, and skin atopy, such as eczema, or respiratory atopy or gingival hypertrophy.
The invention also relates to the compositions as a medicament for the treatment or prevention of the pathologies cited above.
The invention will now be illustrated by the following examples.

### EXAMPLE 1: Solubility of the vitamin D derivative in various excipients

In the table below are the solubility results for (4E,6E)-7-[3-(3,4-bishydroxymethylbenzyloxy)phenyl]-3-ethylnona-4,6-dien-3-ol, a vitamin D analogue, in various excipients:

| Solution | Concentration in % (w/w) |
| --- | --- |
| Propylene glycol | 6.7 |
| Ethanol Rectapur | 23.3 |
| Transcutol | 27.0 |
| Eutanol G | 0.5 |
| Miglyol 812 | 0.4 |
| Tegosoft TN | 0.3 |
| Crodamol IPP | 0.2 |
| PEG 400 | 17.8 |
| pH 4 | < 0.05 |
| pH 7 | < 0.05 |
| Silicone DC 200 | < 0.05 |
| Primol 352 | < 0.05 |
| Marcol 172 | < 0.05 |
| Glycerine | < 0.05 |

(continued)

| Solution | Concentration in % (w/w) |
|---|---|
| Cetiol SN | < 0.1 |

These maximum solubilities of (4E,6E)-7-[3-(3,4-bishydroxymethylbenzyloxy)phenyl]-3-ethylnona-4,6-dien-3-ol, a vitamin D analogue, were measured after stirring for 12 h with a magnetic stirrer bar, at room temperature, with an excess of active ingredient in the excipient to be analysed. The suspension is then filtered (1.2 $\mu$m) and then the filtrate is assayed by HPLC.

**EXAMPLES 2 to 10: Methods of preparation**

In the examples below (Examples 2 to 10), the proportions of the various constituents are expressed as percentages by weight, unless otherwise stated. The active compounds used are the following:

Compound 1: (4E,6E)-7-[3-(3,4-bishydroxymethylbenzyloxy)phenyl]-3-ethylnona-4,6-dien-3-ol;
Compound 2: {5-[6,2'-diethyl-4'-(1-ethyl-1-hydroxypropyl)biphenyl-3-yloxymethyl]-2-hydroxymethylphenyl}methanol;
Compound 3: {4-[6-ethyl-4'-(1-ethyl-1-hydroxypropyl)-2'-propylbiphenyl-3-yloxymethyl]-2-hydroxymethylphenyl}methanol.

The compositions of Examples 2 and 3 are prepared in the following manner:

Fatty phase A

- Weigh the constituents of the fatty phase (A): Mirasil CM5, Primol 352, Cetiol SN, Eumulgin B2, DC Emulsifier 10, butylated hydroxytoluene.
- Heat to 55°C, with stirring using deflocculating device at 200 rpm.

Phase B

- Heat the PEG 400 to 55°C.

- Weigh and introduce the active compound into the PEG 400, with stirring using a magnetic stirrer bar (speed 5).

Pre-emulsification
Slowly introduce the active phase (B) into the fatty phase (A), with stirring at 1000 rpm, and 55°C.
Cooling
Keep stirring (800-1000 rpm) until room temperature (RT) is reached.
Phase C
Solubilize the $MgSO_4$ in water, and with stirring using a magnetic stirrer bar.
Addition of phase D
At RT, add phase D - Ethanol Rectapur - to phase C.
Emulsification
Slowly introduce the aqueous phase (C+D) into the pre-emulsion, with stirring at 1500 rpm.

The compositions of Examples 4 to 9 are prepared in the following manner:

Preparation of Phase A:
The hydrophobic constituents are mixed and heated to 50°C.
Preparation of Phase B1:
The active compound is solubilized in propylene glycol.
Preparation of Phase B2:
The electrolyte ($MgSO_4$ or NaCl) is dissolved in water.
Add Phases B2 and B3 to Phase B1 and heat to 50°C.
Phase B is incorporated into Phase A, with gentle mechanical stirring.

Example 10 is prepared in the following manner:

Preparation of Phase A:
The hydrophobic constituents are mixed and heated to 50°C.
Preparation of Phase B:
Compound 2 is solubilized in propylene glycol at 55°C.
Phase B is incorporated into Phase A, with gentle mechanical stirring at 50°C.
Preparation of Phase C:
The electrolyte is dissolved in water. Next, ethanol is incorporated.
This phase is introduced at room temperature into the emulsion, with gentle stirring.

## Example 2: Composition

Phase A

| | |
|---|---|
| Mirasil CM 5 | 6.00% |
| Primol 352 | 3.00% |
| Cetiol SN | 7.00% |
| Eumulgin B2 | 1.00% |
| DC Emulsifier 10 | 3.00% |
| Butylated hydroxytoluene | 0.10% |

Phase B

| | |
|---|---|
| PEG 400 | 58.40% |
| Compound 3 | 0.30% |

Phase C

| | |
|---|---|
| Purified water | 14.00% |
| Magnesium sulphate.7$H_2$O | 1.00% |

Phase D

| | |
|---|---|
| Ethanol Rectapur | 5.00% |

## Example 3: Composition

Phase A

| | |
|---|---|
| Mirasil CM 5 | 6.00% |

| | |
|---|---|
| Primol 352 | 3.00% |
| Cetiol SN | 7.00% |
| Eumulgin B2 | 1.00% |
| DC Emulsifier 10 | 3.00% |
| Butylated hydroxytoluene | 0.10% |

Phase B

| | |
|---|---|
| Propylene glycol | 58.40% |
| Compound 3 | 0.30% |

Phase C

| | |
|---|---|
| Purified water | 14.00% |
| Magnesium sulphate.7H$_2$O | 1.00% |

Phase D

| | |
|---|---|
| Ethanol Rectapur | 5.00% |

**Example 4: Composition**

Phase A

| | |
|---|---|
| Emulsifier 10 (lauryl methicone copolyol) | 5.00% |
| Cyclomethicone | 15.00% |
| Light paraffin oil | 15.00% |
| Cetostearyl alcohol | 3.00% |

Phase B1

| | |
|---|---|
| Propylene glycol | 19.00% |
| Dipropylene glycol | 32.00% |
| Glycerine | 10.00% |
| Compound 3 | 1.00% |

**Example 5: Composition**

Phase A

| | |
|---|---|
| Emulsifier 10 (lauryl methicone copolyol) | 3.00% |
| Cyclomethicone | 10.00% |
| Paraffin oil | 10.00% |
| Ceteareth-20 | 1.00% |

Phase B1

Propylene glycol    75.00%
Compound 3          1.00%

**Example 6: Composition**

Phase A

Emulsifier 10 (lauryl methicone copolyol)    3.00%
Cyclomethicone                               10.00%
Cetearyl isononanoate                        7.00%
Paraffin oil                                 3.00%
Ceteareth-20                                 1.00%

Phase B1

Propylene glycol    58.00%
Compound 2          2.00%

Phase B2

Water     10.00%
$MgSO_4$  1.00%

Phase B3

Ethanol    5.00%

**Example 7: Composition**

Phase A

Emulsifier 10 (lauryl methicone copolyol)    3.00%
Cyclomethicone                               15.00%
C12-C15 alkyl benzoate                       15.00%

Phase B1

Propylene glycol    56.00%
Compound 1          1.00%

Phase B2

Water    10.00%

**Example 8: Composition**

Phase A

Dimethicone copolyol and cyclomethicone    3.00%
Cyclomethicone                              10.00%
Cetearyl isononanoate                       7.00%
Paraffin oil                                3.00%
Ceteareth-20                                1.00%
Zinc stearate                               1.00%

Phase B1

Propylene glycol    48.00%
Compound 3          1.00%

Phase B2

Water    20.00%
NaCl     1.00%

Phase B3

Ethanol Rectapur    5.00%

**Example 9: Composition**

Phase A

Alkyl methicone copolyol    3.00%
Cyclomethicone              10.00%
Cetearyl isononanoate       7.00%
Paraffin oil                3.00%
Ceteareth-20                1.00%

Phase B1

Propylene glycol    49.30%
Compound 3          1.00%

Phase B2

| | |
|---|---|
| Water | 20.00% |
| $MgSO_4$ | 0.70% |

Phase B3

Ethanol     5.00%

**Example 10: Composition**

Phase A

| | |
|---|---|
| Alkyl methicone copolyol | 3.00% |
| Cyclomethicone | 6.00% |
| Cetearyl isononanoate | 7.00% |
| Paraffin oil | 3.00% |
| Ceteareth-20 | 1.00% |
| BHT | 0.10% |

Phase B

| | |
|---|---|
| Propylene glycol | 58.40% |
| Compound 2 | 1.50% |

Phase C

| | |
|---|---|
| Water | 14.00% |
| Electrolytes | 1.00% |
| Ethanol | 5.00% |

**Claims**

1. Composition containing at least one active agent sensitive to the presence of water, which is not DHEA and/or its chemical and/or biological precursors or derivatives, **characterized in that** the composition is an invert emulsion containing a glycolic or hydroglycolic dispersed hydrophilic phase, a lipophilic continuous phase and an emulsifier having an HLB of between 2 and 7, said active agent being solubilized in said glycolic or hydroglycolic dispersed hydrophilic phase, and said active agent being a derivative of vitamin D chosen from calcitriol, (4E,6E)-7-[3-(3,4-bis-hydroxymethylbenzyloxy)phenyl]-3-ethylnona-4,6-dien-3-ol   and   {4-[6-Ethyl-4'-(1-éthyl-1-hydroxy-propyl)-2'-propyl-biphényl-3-yloxyméthyl]-2-hydroxyméthyl-phényl}-methanol.

2. Composition according to Claim 1, **characterized in that** the emulsifier is a silicone emulsifier.

3. Composition according to Claim 2, **characterized in that** the emulsifier is chosen from lauryl methicone copolyol, cetyl dimethicone copolyol, a mixture of dimethicone copolyol and cyclomethicone or a mixture of cetyl dimethicone copolyol with polyglyceryl-4 isostearate and hexyl laurate.

4. Composition according to one of Claims 1 to 3, **characterized in that** it also contains a coemulsifier having an HLB of greater than 6.

5. Composition according to Claim 4, **characterized in that** the coemulsifier is ceteareth-20.

6. Composition according to one of Claims 1 to 5, **characterized in that** the proportion by volume of glycol, relative to the total volume of the dispersed phase, is between 10 and 100%.

7. Composition according to one of Claims 1 to 6, **characterized in that** the dispersed phase comprises at least one glycol chosen from propylene glycol, hexylene glycol, dipropylene glycol and PEG 400.

8. Composition according to one of Claims 1 to 7, **characterized in that** the water activity of the dispersed hydrophilic phase is less than 0.85.

9. Composition according to one of claims 1 to 8, **characterized in that** the volume fraction of the dispersed hydrophilic phase ranges from 10 to 90% relative to the total volume of the emulsion.

10. Composition according to one of Claims 1 to 9, **characterized in that** it contains between 0.001 and 20% by weight of the active agent sensitive to the presence of water, relative to the total weight of the composition.

11. Composition according to one of Claims 1 to 10, **characterized in that** it contains between 0.01 and 4% by weight of derivatives of vitamin D, relative to the total weight of the composition.

12. Use of a composition according to any one of the preceding claims for the preparation of a medicament intended for the prevention or treatment:

   - of dermatological conditions linked to a keratinocyte or sebocyte differentiation or proliferation disorder;
   - of keratinization disorders;
   - of dermatological conditions linked to a keratinization disorder with an inflammatory and/or immunoallergic component;
   - of inflammatory skin conditions not exhibiting a keratinization disorder;
   - of dermal or epidermal proliferation;
   - of dermatological disorders such as bullous dermatosis and collagen diseases;
   - of the signs of skin ageing, whether photoinduced or chronological, or for reducing actinic pigmentations and keratoses, or any skin pathologies associated with chronological or actinic ageing;
   - of cicatrization disorders and stretch marks;
   - of sebaceous function disorders such as acne hyperseborrhoea or ordinary seborrhoea or seborrhoeic eczema;
   - of dermatological conditions with an immunological component.

13. Use of a composition according to Claim 12, **characterized in that** the dermatological conditions linked to a keratinocyte or sebocyte differentiation or proliferation disorder relate to acne vulgaris, comedo-type acne, polymorphic acne, acne rosacea, nodulocystic acne, acne conglobata, senile acne, secondary acne such as solar acne, acne medicamentosa or occupational acne.

14. Use according to Claim 12, **characterized in that** the keratinization disorders relate to ichthyosis, ichthyosiform states, Darier's disease, keratosis palmaris et plantaris, leukoplakia and leukoplakia-like states, or skin or mucosal (buccal) lichen.

15. Use according to Claim 12, **characterized in that** the dermatological conditions linked to a keratinization disorder with an inflammatory and/or immunoallergic component relate to all forms of psoriasis, whether continuous, mucosal or ungual, arthropathic psoriasis, and skin atopy, such as eczema, or respiratory atopy or gingival hypertrophy.

16. Use according to Claim 12, **characterized in that** the dermal or epidermal proliferations may be benign or malignant, of non-viral origin or of viral origin, such as verruca vulgaris, verruca plana and epidermodysplasia verruciformis, and oral or florid papillomatosis, and **in that** the proliferations may be induced by ultraviolet radiation, in particular in the case of baso- and spinocellular epithelioma.

17. Composition according to one of Claims 1 to 11, as a medicament.

**Patentansprüche**

1. Zusammensetzung, die mindestens einen Wirkstoff enthält, der gegen vorliegendes Wasser empfindlich ist und bei dem es sich nicht um DHEA oder seine chemischen und/oder biologischen Vorläufer oder Derivate handelt, **dadurch gekennzeichnet, dass** die Zusammensetzung eine Umkehremulsion ist, die eine hydrophile disperse Glycolphase oder wässerige Glycolphase, eine lipophile kontinuierliche Phase sowie einen Emulgator mit einem HLB-Wert im Bereich von 2 bis 7 enthält, wobei der Wirkstoff in der hydrophilen dispersen Glycolphase oder wässerigen Glycolphase solubilisiert ist und wobei der Wirkstoff ein Derivat von Vitamin D ist, das ausgewählt ist unter Calcitriol, (4E,6E)-7-[3-(3,4-Bis-hydroxy-methylbenzyloxy)-phenyl]-3-ethylnona-4,6-dien-3-ol und {4-[6-Ethyl-4'-(1-ethyl-1-hydroxy-propyl)-2'-propyl-biphenyl-3-yloxymethyl]-2-hydroxymethyl-phenyl}-methanol.

2. Zusammensetzung nach Anspruch 1, **dadurch gekennzeichnet, dass** der Emulgator ein Siliconemulgator ist.

3. Zusammensetzung nach Anspruch 2, **dadurch gekennzeichnet, dass** der Emulgator ausgewählt ist unter Laurylmethicon-Copolyol, Cetyldimethicon-Copolyol, einem Gemisch von Dimethicon-Copolyol und Cyclomethicon oder einem Gemisch von Cetyldimethicon-Copolyol mit Polyglyceryl-4-isostearat und Hexyllaurat.

4. Zusammensetzung nach einem der Ansprüche 1 bis 3, **dadurch gekennzeichnet, dass** sie ferner einen Coemulgator mit einem HLB-Wert von mehr als 6 enthält.

5. Zusammensetzung nach Anspruch 4, **dadurch gekennzeichnet, dass** der Coemulgator Ceteareth-20 ist.

6. Zusammensetzung nach einem der Ansprüche 1 bis 5, **dadurch gekennzeichnet, dass** der volumenbezogene Mengenanteil an Glycol, bezogen auf das Gesamtvolumen der dispersen Phase, im Bereich von 10 bis 100 % liegt.

7. Zusammensetzung nach einem der Ansprüche 1 bis 6, **dadurch gekennzeichnet, dass** die disperse Phase mindestens ein Glycol enthält, das unter Propylenglycol, Hexylenglycol, Dipropylenglycol und PEG 400 ausgewählt ist.

8. Zusammensetzung nach einem der Ansprüche 1 bis 7, **dadurch gekennzeichnet, dass** die Wasseraktivität der dispersen hydrophilen Phase weniger als 0,85 beträgt.

9. Zusammensetzung nach einem der Ansprüche 1 bis 8, **dadurch gekennzeichnet, dass** der Volumenanteil der dispersen hydrophilen Phase im Bereich von 10 bis 90 % liegt, bezogen auf das Gesamtvolumen der Emulsion.

10. Zusammensetzung nach einem der Ansprüche 1 bis 9, **dadurch gekennzeichnet, dass** sie 0,001 bis 20 Gew.-% gegen vorliegendes Wasser empfindlichen Wirkstoff enthält, bezogen auf das Gesamtgewicht der Zusammensetzung.

11. Zusammensetzung nach einem der Ansprüche 1 bis 10, **dadurch gekennzeichnet, dass** sie 0,01 bis 4 Gew.-% Derivate von Vitamin D enthält, bezogen auf das Gesamtgewicht der Zusammensetzung.

12. Verwendung einer Zusammensetzung nach einem der vorhergehenden Ansprüche zur Herstellung eines Arzneimittels zur Vorbeugung oder zur Behandlung von

    - dermatologischen Zuständen, die mit einer Störung oder Differenzierung oder Proliferation von Keratinocyten oder Sebocyten in Zusammenhang stehen;
    - dermatologischen Zuständen, die mit einer Störung der Keratinisierung in Zusammenhang stehen und eine entzündliche und/oder immunoallergische Komponente aufweisen;
    - entzündlichen Hautzuständen, bei denen keine Störung der Keratinisierung vorliegt;
    - der dermalen oder epidermalen Proliferation;
    - dermatologischen Störungen, wie etwa der bullösen Dermatose sowie von Collagen-Erkrankungen;
    - Zeichen der Hautalterung, die lichtinduziert oder altersbedingt sein kann, oder zur Verringerung strahlenbedingter Pigmentationen und Keratosen oder beliebiger Hautsymptomatiken, die mit altersbedingter oder strahlenbedingter Alterung in Verbindung stehen;
    - Störungen der Narbenbildung sowie von Schwangerschaftsstreifen;
    - Störungen der Talgdrüsenfunktion wie etwa Acne hyperseborrhoea oder gewöhnlicher Seborrhoea oder von seborrhoischem Ekzem;
    - dermatologischen Zuständen mit einer immunologischen Komponente.

**13.** Verwendung einer Zusammensetzung nach Anspruch 12, **dadurch gekennzeichnet, dass** die dermatologischen Zustände, die mit einer Störung der Differenzierung oder Proliferation von Keratinocyten oder Sebocyten in Zusammenhang stehen, sich auf Acne vulgaris, Akne vom Comedo-Typ, polymorphe Akne, Acne rosacea, nodulocystische Akne, Acne conglobata, senile Akne, sekundäre Akne, wie etwa Sonnenakne, Acne medicamentosa oder Berufsakne beziehen.

**14.** Verwendung nach Anspruch 12, **dadurch gekennzeichnet, dass** sich die Störungen der Keratinisierung auf Ichthyosis, ichthyosiforme Zustände, Darier-Krankheit, Keratosis palmaris und Keratosis plantaris, Leukoplakie und Leukoplakie-ähnliche Zustände oder Lichen der Haut oder der Schleimhäute (buccal) beziehen.

**15.** Verwendung nach Anspruch 12, **dadurch gekennzeichnet, dass** die mit einer Störung der Keratinisierung mit einer entzündlichen und/oder immunoallergischen Komponente in Zusammenhang stehenden dermatologischen Zustände sich auf sämtliche Formen der Psoriasis, unabhängig davon, ob es sich um kontinuierliche, mucosale oder unguale oder arthropathische Psoriasis handelt, sowie Hautatopie, wie Ekzem, oder respiratorische Atopie oder gingivale Hypertrophie beziehen.

**16.** Verwendung nach Anspruch 12, **dadurch gekennzeichnet, dass** die dermalen oder epidermalen Proliferationen benigne oder maligne Proliferationen nichtviralen oder viralen Ursprungs sein können, wie etwa Verruca vulgaris, Verruca plana und Epidermodysplasia verruciformis, sowie orale oder floride Papillomatosen, sowie, dass die Proliferationen durch Ultraviolettstrahlung induziert sein können, insbesondere im Fall von basocellulären und spinocellulären Epitheliomen.

**17.** Zusammensetzung nach einem der Ansprüche 1 bis 11 als Arzneimittel.

**Revendications**

**1.** Composition contenant au moins un agent actif sensible à la présence d'eau, qui n'est pas la DHEA et/ou ses précurseurs ou dérivés chimiques et/ou biologiques, **caractérisée en ce que** la composition est une émulsion inverse contenant une phase hydrophile dispersée glycolique ou hydroglycolique, une phase continue lipophile et un émulsionnant de HLB compris entre 2 et 7, ledit agent actif étant solubilisé dans ladite phase hydrophile dispersée glycolique ou hydroglycolique et ledit agent actif étant un dérivé de vitamine D choisi parmi le calcitriol, le (4E,6E)-7-[3-(3,4-bis-hydroxyméthylbenzyloxy)phényl]-3-éthylnona-4,6-dién-3-ol et le {4-[6-éthyl-4'-(1-éthyl-1-hydroxypropyl)-2'-propyl-biphényl-3-yloxyméthyl]-2-hydroxyméthyl-phényl}-méthanol.

**2.** Composition selon la revendication 1, **caractérisée en ce que** l'émulsionnant est un émulsionnant siliconé.

**3.** Composition selon la revendication 2, **caractérisée en ce que** l'émulsionnant est choisi parmi le lauryl méthicone copolyol, le cétyl diméthicone copolyol, un mélange de diméthicone copolyol et de cyclométhicone et un mélange de cétyl diméthicone copolyol avec du polyglycéryl-4 isostéarate et du laurate d'hexyle.

**4.** Composition selon l'une des revendications 1 à 3, **caractérisée en ce qu'**elle contient également un co-émulsionnant dont le HLB est supérieur à 6.

**5.** Composition selon la revendication 4, **caractérisée en ce que** le co-émulsionnant est le cétéareth-20.

**6.** Composition selon l'une des revendications 1 à 5, **caractérisée en ce que** la proportion volumique de glycol, par rapport au volume total de la phase dispersée, est comprise entre 10 et 100 %.

**7.** Composition selon l'une des revendications 1 à 6, **caractérisée en ce que** la phase dispersée comprend au moins un glycol choisi parmi le propylène glycol, l'hexylène glycol, le dipropylène glycol et le PEG 400.

**8.** Composition selon l'une des revendications 1 à 7, **caractérisée en ce que** l'activité en eau de la phase hydrophile dispersée est inférieure à 0,85.

**9.** Composition selon l'une des revendications 1 à 8, **caractérisée en ce que** la fraction volumique de la phase hydrophile dispersée se situe entre 10 et 90 % par rapport au volume total de l'émulsion.

**10.** Composition selon l'une des revendications 1 à 9, **caractérisée en ce qu'**elle contient entre 0,001 et 20 % en poids de l'agent actif sensible à la présence d'eau, par rapport au poids total de la composition.

**11.** Composition selon l'une des revendications 1 à 10, **caractérisée en ce qu'**elle contient entre 0,01 et 4 % en poids de dérivés de vitamine D, par rapport au poids total de la composition.

**12.** Utilisation d'une composition selon l'une quelconque des revendications précédentes, pour la préparation d'un médicament destiné à la prévention ou au traitement :

- des affections dermatologiques liées à un trouble de la différenciation ou de la prolifération des kératinocytes ou des sébocytes ;
- des troubles de la kératinisation ;
- des affections dermatologiques liées à un trouble de la kératinisation à composante inflammatoire et/ou immuno-allergique ;
- des affections inflammatoires cutanées ne présentant pas de trouble de la kératinisation ;
- de la prolifération dermique ou épidermique ;
- des troubles dermatologiques tels que la dermatose bulleuse et les maladies du collagène ;
- des signes du vieillissement de la peau, qu'il soit photo-induit ou chronologique, ou à la réduction des pigmentations et des kératoses actiniques, ou d'une quelconque pathologie cutanée associée au vieillissement chronologique ou actinique ;
- des troubles de la cicatrisation et des vergetures ;
- des troubles de la fonction sébacée tels que l'hyperséborrhée de l'acné ou la séborrhée simple ou l'eczéma séborrhéique ;
- des affections dermatologiques à composante immunologique.

**13.** Utilisation d'une composition selon la revendication 12, **caractérisée en ce que** les affections dermatologiques liées à un trouble de la différenciation ou de la prolifération des kératinocytes ou des sébocytes concernent l'acné vulgaire, l'acné comédonienne, l'acné polymorphe, l'acné rosacée, l'acné nodulokystique, l'acné conglobata, l'acné sénile, l'acné secondaire telle que l'acné solaire, l'acné médicamenteuse ou l'acné professionnelle.

**14.** Utilisation selon la revendication 12, **caractérisée en ce que** les troubles de la kératinisation concernent l'ichtyose, les états ichtyosiformes, la maladie de Darier, la kératose palmoplantaire, la leucoplasie et les états leucoplasiformes, ou le lichen cutané ou muqueux (buccal).

**15.** Utilisation selon la revendication 12, **caractérisée en ce que** les affections dermatologiques liées à un trouble de la kératinisation à composante inflammatoire et/ou immuno-allergique concernent toutes les formes de psoriasis, qu'il soit cutané, muqueux ou unguéal, le psoriasis arthropatique et l'atopie cutanée telle que l'eczéma, ou l'atopie respiratoire ou l'hypertrophie gingivale.

**16.** Utilisation selon la revendication 12, **caractérisée en ce que** les proliférations dermiques ou épidermiques peuvent être bénignes ou malignes, d'origine non virale ou d'origine virale, telles que les verrues vulgaires, les verrues planes et l'épidermodysplasie verruciforme, et la papillomatose orale ou floride, et **en ce que** les proliférations peuvent être induites par les rayonnements ultraviolets, en particulier dans le cas des épithéliomas baso- et spinocellulaires.

**17.** Composition selon l'une des revendications 1 à 11, à titre de médicament.

**EP 1 592 452 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0779071 A

- WO 0026167 A

**Non-patent literature cited in the description**

- **MOTOYOSHI et al.** *Cosmet. and toiletries,* 1984, vol. 99, 83-89

- *S. paint Technology,* 1967, vol. 30, 195